# EUROPEAN PATENT SPECIFICATION

(11) **EP 3 047 826 B1**
(45) Date of publication and mention of the grant of the patent: **14.03.2018**
(21) Application number: 13888643.7
(22) Date of filing: 25.09.2013
(51) Int. Cl.: A61F 13/15

(54) **URINE ABSORPTION PAD FOR MEN**
URINABSORPTIONSKISSEN FÜR MÄNNER
GARNITURE DE COLLECTE D'URINE POUR HOMME

(30) Priority: 20.09.2013 JP 2013196229
(43) Date of publication of application: 27.07.2016
(73) Proprietor: Unicharm Corporation, Ehime 799-0111 (JP)
(72) Inventor: FUJIMOTO, Kazuya, Kanonji-shi Kagawa 769-1602 (JP); NAKAJIMA, Kaiyo, Kanonji-shi Kagawa 769-1602 (JP)
(74) Representative: Peter, Julian
(86) International application number: PCT/JP2013/075805
(87) International publication number: WO 2015/001685

(56) References cited:
- WO-A1-92/15269
- JP-A- H09 506 280
- JP-A- 2008 237 391
- JP-A- 2009 006 019
- US-A1- 2013 090 621
- US-B1- 6 371 950

## Description

### {Technical Field}

The present invention relates to male urine absorption pads.

### {Background}

Conventionally, the male urine absorption pads are known. For example, a male urine absorption pad disclosed in undermentioned Patent Literature 1 includes a pad main body having liquid-absorbability, an opening defined by an elastic barrier-flap so that a wearer's sexual organ may be inserted through the opening, and a 3D-space to receive the wearer's sexual organ.

### {Citation List}

### {Patent Literature}

{PTL 1}: JUM H8-1058

### {Summary}

### {Technical Problem}

In terms of the disclosure of the male urine absorption pad in Patent Literature 1, the sexual organ inserted through the opening (insertion opening) defined by the elastic barrier-flaps are elastically tightened to assure a high fit between the wearer's sexual organ and the barrier-flaps. In this way, a gap causing the leakage of body exudates may be left between the wearer's sexual organ and the barrier-flaps.

However, the barrier-flaps include elastic element arranged in a region thereof defining a peripheral edge of the opening and, when the wearer impresses a force in a lateral direction upon the pad held in his hands to put it on body, the opening may become reduced in size under contraction of the elastic element and make it difficult to put the pad on his body. With the pad put on the wearer's body, the barrier-flaps may also be wedged between the thighs and consequently the barrier-flaps may be elastically deformed until it becomes difficult for the opening to maintain a size and a shape required to ensure a 3D-space sufficient to receive and absorb the urine.

An object of the present invention is to improve such a pad of the prior art, thereby providing a male urine absorption pad formed with a 3D-space having a sufficient capacity for temporary storage of body exudates and reliably preventing the body exudates from leaking out.

### {Solution to Problem}

The present invention to solve the problem set forth is directed to a male urine absorption pad having a longitudinal direction, a transverse direction being orthogonal thereto, a skin-facing surface and a non-skin-facing surface opposed thereto and including a pad main body having a liquid-absorbent core,

The male urine absorption pad according to the present invention is featured to the pad main body that has first and second end regions opposite to each other in the longitudinal direction, both lateral regions opposite to each other in the transverse direction and a central region defined between both the lateral regions; of the first and second end regions, at least the first end region is provided with a cover sheet adapted to cover the central region and both the lateral regions are provided with a pair of barrier-flaps extending in the longitudinal direction; each of the barrier-flaps has both end portions fixed to the skin-facing surface side of the pad main body and free edge portion extending in the longitudinal direction between both the fixed end portions; each of the free edge portions has an elastic region being contractible in the longitudinal direction and inelastic regions located on the outer side of the elastic region as viewed in the longitudinal direction; the cover sheet is formed of inelastic sheet material and has inner edge portion extending across the central region, both side edge portions fixed to the barrier-flaps in the first end region, a central portion fixed to the pad main body located between both the side edge portions and a covering portion defined between both the side edge portions and the central portion to cover the central region; a urine-receivable 3D-space having an opening edge portion defined by the inner side edge portion of the cover sheet is formed between the covering portion and the pad main body; and a contractile action of the elastic region is exerted to the opening edge portions.

### {Advantageous Effects of Invention}

According to one or more embodiments of the present invention, the cover sheet fixed to the barrier-flaps is spaced apart upward as the free edge portion of the barrier-flaps arises and, in consequence, a 3D-space having a sufficient volume to receive the body exudates is formed between the cover sheet and the pad main body. The elastic region of the free edge portion extending to the opening's end makes it possible to form a relatively large opening. Further, the cover sheet having no elasticity makes it possible to avoid a likelihood that the cover sheet may be deformed to reduce a volume of the 3D-space even when the portion inclusive of the cover sheet is wedged between the wearer's thighs.

### {Brief Description of Drawings}

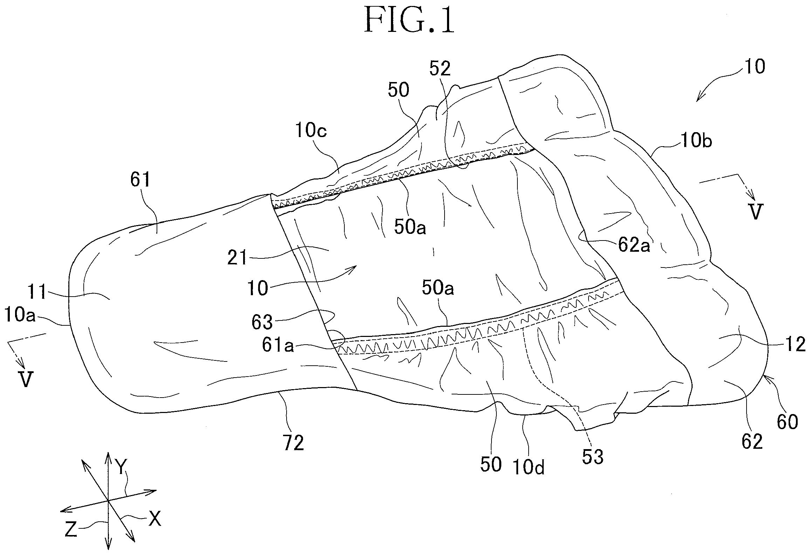

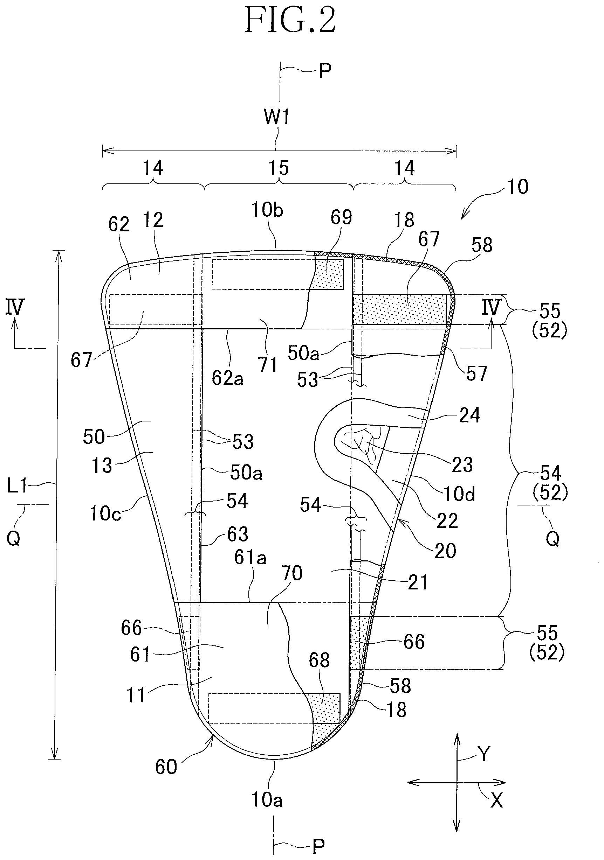

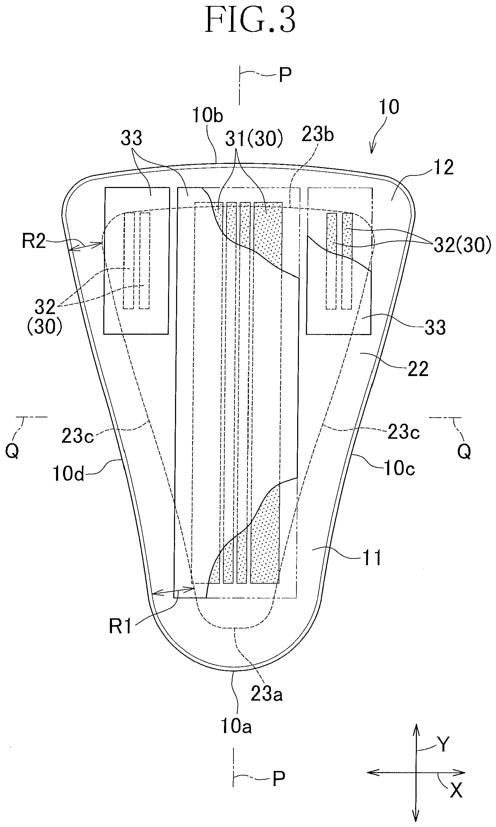

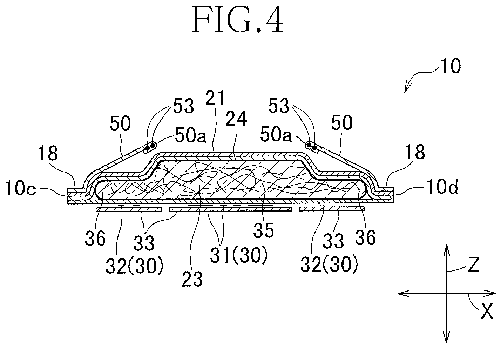

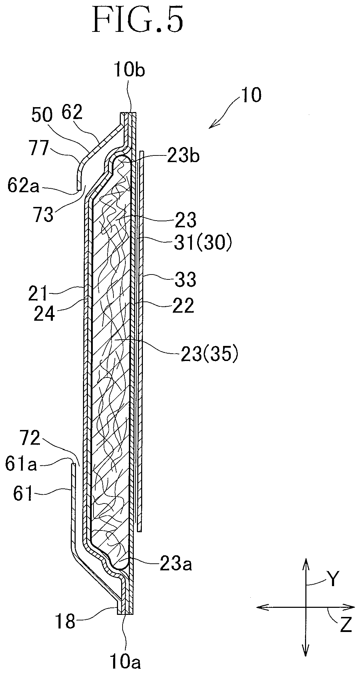

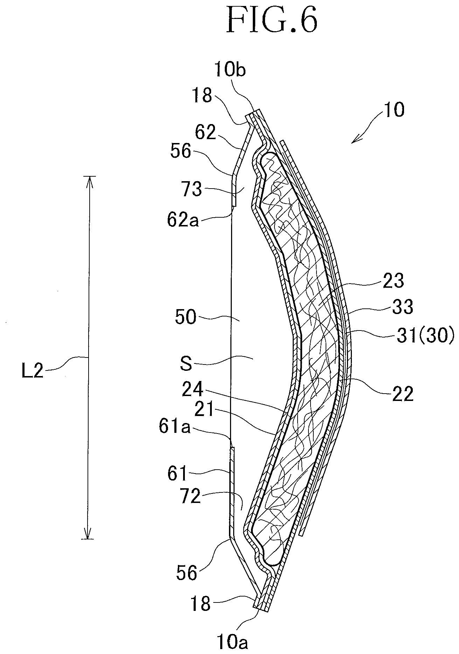

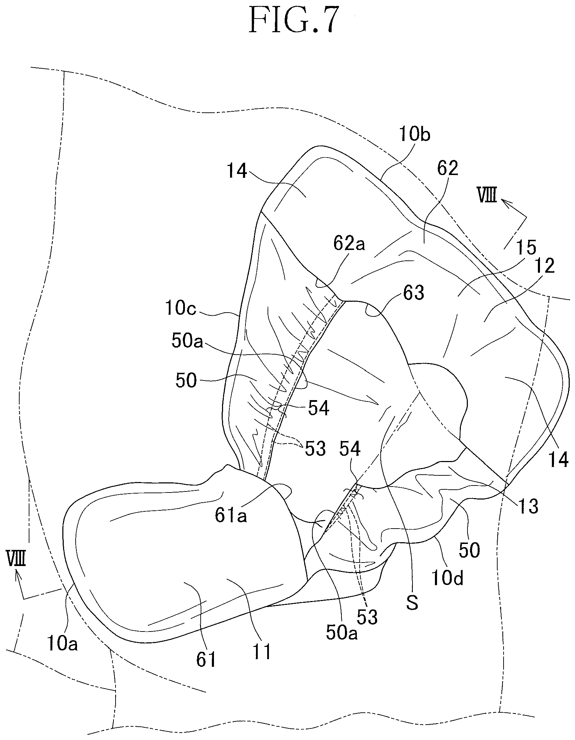

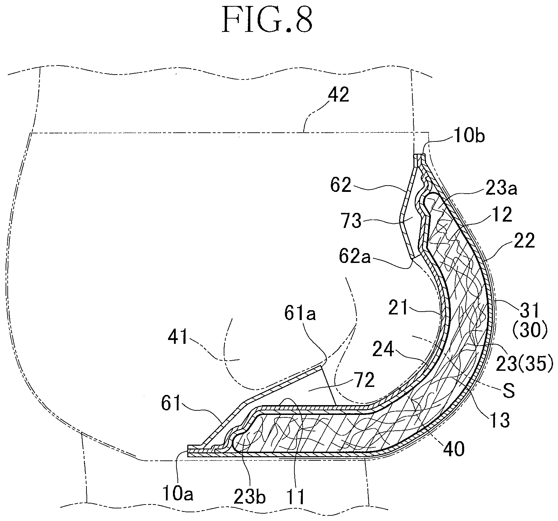

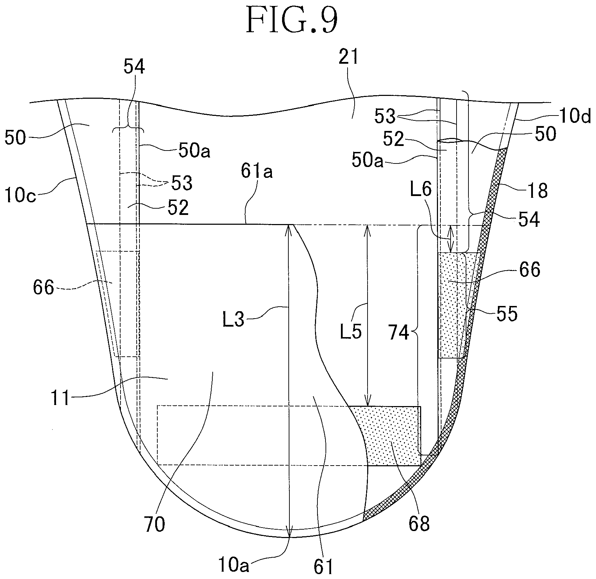

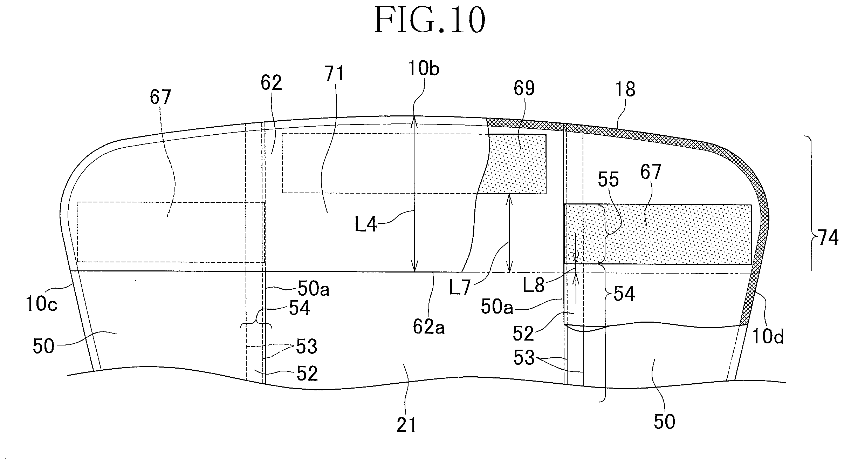

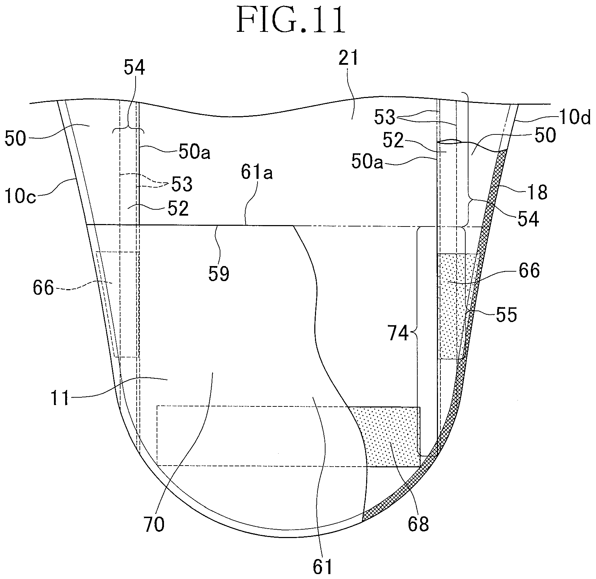

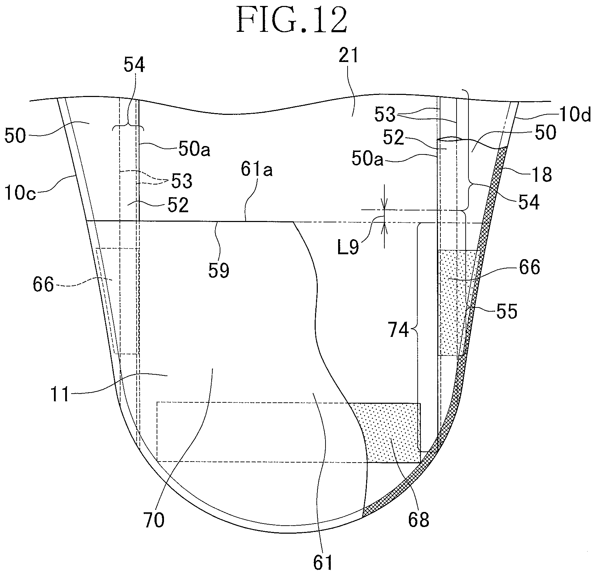

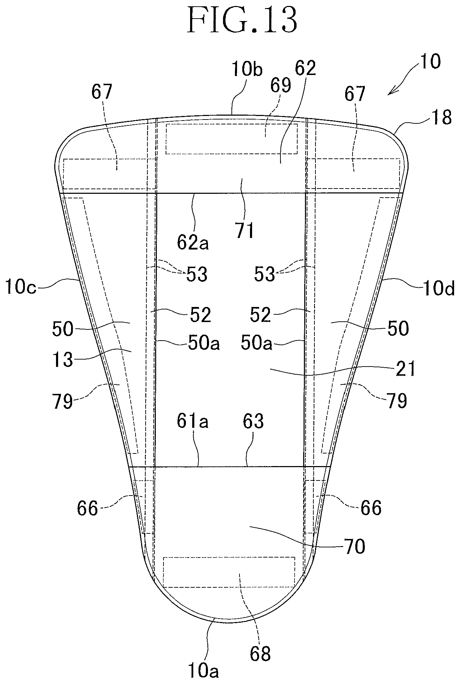

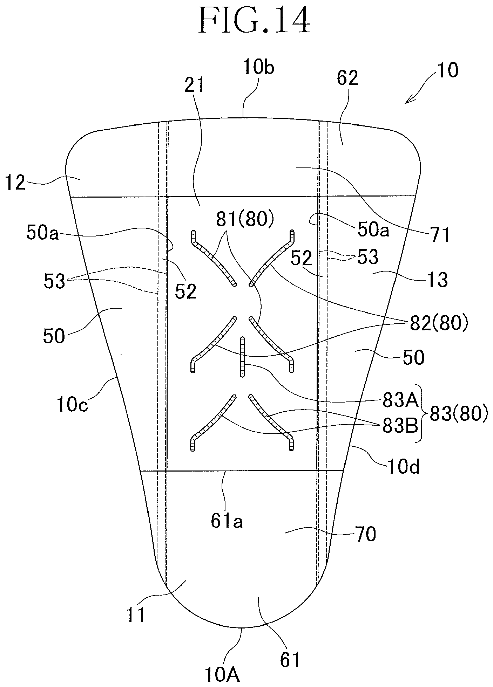

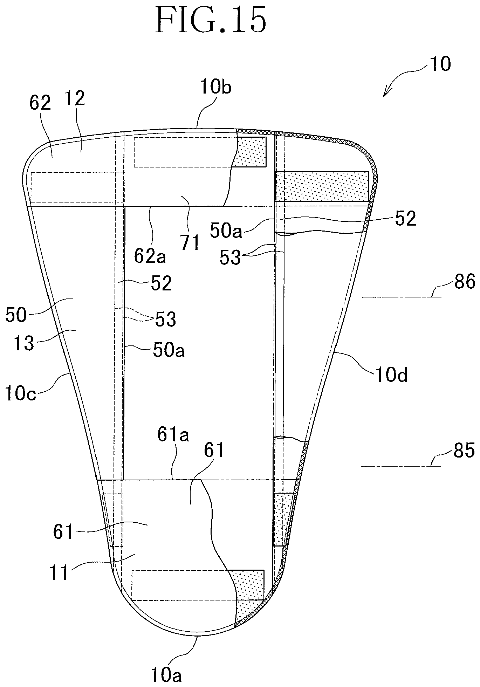

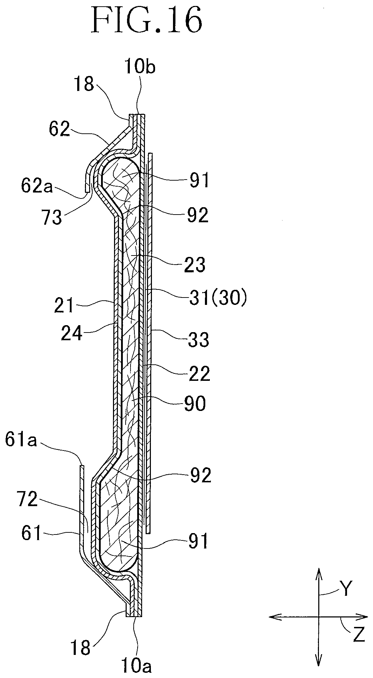

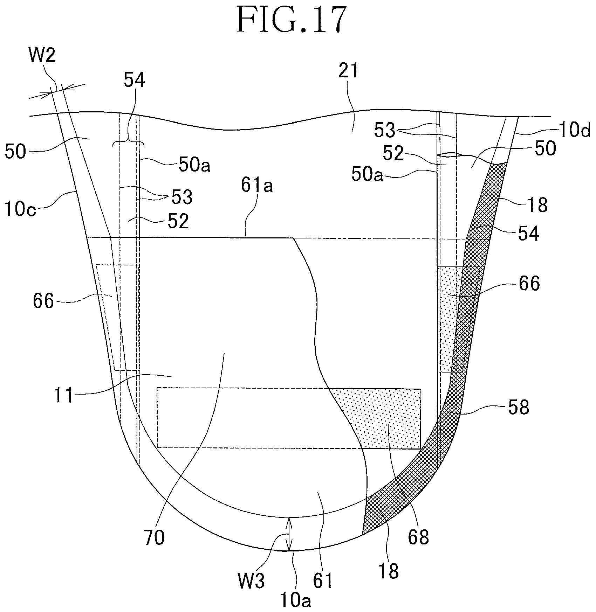

The drawings illustrate including optional and preferred embodiments as well as essential features of the inventions:
{Fig. 1} A perspective view of a male urine absorption pad according to a first embodiment of the present invention.
{Fig. 2} A partially cutaway plan view of the urine absorption pad according to the first embodiment.
{Fig. 3} A partially cutaway plan view of the urine absorption pad as viewed from an underside surface.
{Fig. 4} A schematic sectional view taken along line IV-IV in Fig. 2.
{Fig. 5} A schematic sectional view taken along line V-V in Fig. 1.
{Fig. 6} A schematic sectional view of the urine absorption pad under a contractile force of the cuff's elastic element.
{Fig. 7} A perspective view of the urine absorption pad put on the wearer's body as viewed from the interior side.
{Fig. 8} A schematic sectional view taken along a line VIII-VIII in Fig. 7.
{Fig. 9} A partially enlarged view of a first end region side in Fig. 2.
{Fig. 10} A partially enlarged view of a second end region side in Fig. 2.
{Fig. 11} A view similar to Fig. 9, illustrating an alternative of the urine absorption pad according to the first embodiment.
{Fig. 12} A view similar to Fig. 9 illustrating another alternative of the urine absorption pad according to the first embodiment.
{Fig. 13} A plan view of the urine absorption pad according to a second embodiment.
{Fig. 14} A plan view of the urine absorption pad according to a third embodiment,
{Fig. 15} A plan view of the urine absorption pad according to a fourth embodiment.
{Fig. 16} A schematic sectional view similar to Fig. 5 in a fifth embodiment.
{Fig. 17} A view similar to Fig. 9 in a sixth embodiment.

### {Description of Embodiments}

The embodiments described below is directed to the male urine absorption pad illustrated in Figs. 1 through 17 including optional and preferred arrangements as well as essential arrangements of the invention.

### <First Embodiment>

Figs. 1 through 5 illustrate an example of a male urine absorption pad 10 according to the present invention. In Figs. 1 through 5, the pad 10 is illustrated in a state not under a contractile force of an after-mentioned flap elastic element 53 (i.e., in a relaxed state). The urine absorption pad 10 has a longitudinal direction Y extending in parallel with a longitudinal axis P bisecting a dimension in a transverse direction X of the urine absorption pad 10, the transverse direction X extending in parallel with a transverse axis Q, a thickness direction Z orthogonal to the longitudinal direction Y and the transverse direction X, respectively, and a skin-facing surface and a non-skin-facing surface opposed to the skin-facing surface. The urine absorption pad 10 is shaped symmetrically about the longitudinal axis P and defined by curved first and second end edges 10a, 10b facing opposite to each other in the longitudinal direction Y and both side edges 10c, 10d extending in the longitudinal direction Y between the first and second end edges 10a, 10b.

The urine absorption pad 10 has a first end region 11 defined on the side of the first end edge 10a, a second end region 12 defined on the side of the second end edge 10b and an intermediate region 13 defined between these first and second end regions 11, 12. The second end edge 10b is more gently curved than the first end edge 10a and has a relatively larger dimension in the transverse direction X so that the second end region 12 may have a width larger than that of the first end region 11. As long as advantageous effects of the present invention as described later are assured, the urine absorption pad 10 is not limited to such a shape in planar view and may have various known shapes in planar view, for example, circular or rectangular shape in planar view. Referring to Fig. 8, the first end region 11 is put in contact with the wearer's crotch region (on the lower side of scrotum 41), the second end region 12 is put in contact with the wearer's lower abdominal region and the intermediate region 13 is put in contact with the wearer's penis 40.

The urine absorption pad 10 includes a pad main body 20 defining a general shape of the pad, a pair of barrier-flaps 50 located on the skin-facing surface of the pad main body 20 in both lateral regions 14 thereof and a sheet 60 fixed to the skin-facing surface of the barrier-flaps 50. The cover sheet 60 includes a first cover sheet 61 located in the first end region 11 to cover the portion of the pad main body 20 defined between the barrier-flaps 50 and a second cover sheet 62 located in the second end region 12 to cover the portion of the pad main body 20 defined between the barrier-flaps 50. The pad main body 20, the respective barrier-flaps 50 and the respective cover sheets 60 are layered one on another and fixed in this state along a linear sealing portion 18 formed so as to extend along a peripheral edge of the urine absorption pad 10. The urine absorption pad 10 further includes a pair of side flaps defined by respective portions of the sheets 50, 60 extending outward beyond a peripheral edge of a liquid-absorbent core 23 in the pad main body 20 and joined to each other along the linear sealing portion 18 so as to extend in the longitudinal direction Y on the outer side in the transverse direction X of respective side edges 23c, 23c of the liquid-absorbent core 23 and a pair of end flaps extending in the transverse direction X on the outer side in the longitudinal direction Y of respective end edges 23a, 23b.

### <pad main body>

A dimension L1 in the longitudinal direction Y of the pad main body 20 ranges about 160 mm to about 350 mm and a dimension W1 in the transverse direction X (i.e., a dimension in the transverse direction X of the second end edge 10b) ranges 160 to 300 mm. The pad main body 20 is zoned in the longitudinal direction Y, as a matter of convenience for explanation, into the first and second end regions 11, 12 and the intermediate region 13 and into both the lateral regions 14 and a central region 15 in the transverse direction X. The term "lateral regions" used herein at least means the regions extending outward in the lateral direction X beyond respective inner side edges 50a of the respective barrier-flaps 50 and the terms "first and second end regions 11, 12" respectively mean the regions covered with the first and second cover sheets 61, 62.

The pad main body 20 includes a topsheet 21 lying on the side of the skin-facing surface, a backsheet 22 lying on the side of the non-skin-facing side and the liquid-absorbent core 23 interposed between the topsheet 21 and the backsheet 22. A cushion sheet 24 is optionally interposed between the topsheet 21 and the liquid-absorbent core 23.

The topsheet 21 and the cushion sheet 24 may be formed of, for example, liquid-permeable fibrous nonwoven fabrics and, as such fibrous nonwoven fabrics, various types of known fibrous nonwoven fabrics such as hydrophilic spun bond fibrous nonwoven fabrics or pointbond fibrous nonwoven fabrics each having a mass per unit area in a range of about 15 to about 40 g/m² may be used. The backsheet 2 may be formed of hardly-liquid-permeable or liquid-impermeable fibrous nonwoven fabrics, air-permeable plastic films or laminate sheets thereof and, to form the backsheet 22 from fibrous nonwoven fabrics, for example, spun bond/melt blown/spun bond (SMS) fibrous nonwoven fabrics, spun bond fibrous nonwoven fabric or meltblown fibrous nonwoven fabrics each having a mass per unit area in a range of about 10 to about 30 g/m² may be used.

The cushion sheet 24 is more bulky than the topsheet 21 so that the cushioning properties thereof may be improved and a coloring element being visible through the topsheet 21 may be optionally located in the central region 15 in which the topsheet interposed between the barrier-flaps 50 is exposed. Such coloring element located in the central region 15 of the cushion sheet 24 will make it easy for the wearer to position his penis in contact with the central region 15 when the wearer tries to put his penis in contact with the interior surface of the pad main body 20. In this regard, the material for the cushion sheet 24 preferably has, in addition to the cushioning function, a function to inhibit the urine once having been absorbed in the side of the topsheet 21 flowing back.

The liquid-absorbent core 23 has substantially the same shape as that of the pad main body 20 and includes absorbent material formed of a mixture of wood fluff pulp, superabsorbent polymer particles (SAP) and, optionally, thermoplastic fibers and a liquid-diffusive sheet such as tissue paper used to wrap the core material.

Referring to Fig. 3, a plurality of linear attachment regions 30 formed of pressure-sensitive adhesive and extending in the longitudinal direction Y in which the urine absorption pad 10 is attached to a garment such as the underwear are arranged on the underside surface, i.e., the exterior surface (the non-skin-facing surface) of the urine absorption pad 10. The linear attachment region 30 include a central attachment region 31 extending from the first end region 11 to the second end region 12 across the lateral axis Q, and both lateral attachment regions 32 symmetrically arranged about the central attachment region 31 on the side of the second end region 12. The central attachment region 31 and both the lateral attachment regions 32 are respectively covered with separators 33 made of plastic or fibrous nonwoven fabrics.

Referring to Fig. 4, the liquid-absorbent core 23 has a central region 35 defined by its central portion as viewed in the lateral direction X and lateral regions 36 defined on both sides of the central region 35 as viewed in the lateral direction X. The thickness of the liquid-absorbent core 23 in the central region 35 thereof is larger than that in the lateral regions 36 and, in the vicinity of respective boundaries between the central region 35 and the respective lateral regions 36, a thickness of the liquid-absorbent core 23 gradually increases from the lateral regions 36 to the central region 35. The manner of shaping the liquid-absorbent core 23 in which the central region 35 is thicker than the both lateral regions 36 and consequently the mass per unit area in the central regions 35 is larger than that in the respective lateral regions 36 makes it possible for the central region 35 to exert a liquid-absorbing ability higher than that of the respective lateral regions 36.

The shape of the liquid-absorbent core 23 in which a thickness in the central region 35 and a thickness in the respective lateral regions 36 are relatively different from each other may be obtained, for example, with use of methods as follows: (1) the core material is deposited into a mold cavity having a shape similar to that of the liquid-absorbent core until the cavity is filled with the core material; (2) the mold having the shape similar to that of the liquid-absorbent core 23 is pressed against the core material having an appropriate thickness until the latter is compressed to the desired thickness; (3) a mold designed so that a relatively higher pressure may be applied to portions of the core material corresponding to the lateral regions 36 of the liquid-absorbent core 23 is used; and (4) the core material is shaped as two separate layers of which the one has a relatively large width dimension and the other has a relatively small width dimension and then the small width dimensioned layer is integrally stacked on the central region of the large width dimensioned layer. According to the methods (1) and (4), it is possible to even up densities (g/cm³) of the central region 35 and the respective lateral regions 36, at least substantially. According to the methods (2) and (3), it is possible to set the density (g/cm³) of the respective lateral regions 36 to a level higher than that of the central region 35. In this regard, it is also possible, if necessary, to set a mass per unit area (g/m²) of the central region 35 to be higher than that of the respective lateral regions 36.

Referring to Fig. 5, the liquid-absorbent core 23 is shaped so as to be relatively thin in both the end edge portions 23a, 23b thereof in the longitudinal direction Y. As previously described, the liquid-absorbent core 23 is shaped so that both the lateral regions 36 of the liquid-absorbent core 23 also are shaped so as to be thinner than the central region 35 (See Fig. 4). For such shape, the peripheral edge of the liquid-absorbent core 23 as a whole is relatively thinner, making it possible to restrict an acute change of the thickness (causing a split-level) from the peripheral edge to the end flaps and the side flaps being contiguous to the peripheral edge. In this way, the contour of the liquid-absorbent core 23 should not convexly appear on the exterior surface of the garment to which the urine absorption pad 10 is attached. It is assured thus the peripheral edge portion of the urine absorption pad 10 is kept in close contact with the wearer's body with good fit.

### <barrier-flaps>

Each of the barrier-flaps 50 has a shape extending in the longitudinal direction Y so as to be gradually tapered from the second end edge 10b to the first end edge 10a and may be formed from hardly-liquid-permeable or liquid-impermeable fibrous nonwoven fabrics, for example, spun bond fibrous nonwoven fabrics having a mass per unit area of about 20 g/m². The barrier-flap 50 includes a proximal edge portion 57 (See Fig. 2) extending along both the side edges 10c, 10d and fixed to the pad main body 20, both end portions 58 fixed to the pad main body 20 in the first and second end regions 11, 12 and a sleeve-like free edge portion (distal edge portion) 52 formed by inwardly folding-back an inner side edge of the barrier-flap 50 extending in the longitudinal direction Y between the fixed both end portions 58. The proximal edge portion 57 and the fixed both end portions 58 are defined by a part of the linear sealing portion 18 and, in other words, the entire periphery of the barrier-flap 50 except the free edge portion 52 is fixed to the pad main body 20 along the linear sealing portion 18.

The linear flap elastic element (elastically contractible member) 53 extending in the longitudinal direction Y is attached under tension but in a contractible manner to the free edge portion 52 of the barrier-flap 50. In the pad put on the wearer's body, the respective free edge portions 52 are spaced apart from the skin-facing surface of the topsheet under a constrictive effect of the respective flap elastic elements 53 and, in this manner, a pair of barrier-flaps (upstanding flap) functioning to prevent the urine from leaking sideways. As the flap-elastic element, it is possible to use the elastic material having a fineness (diameter of string or strand) of about 940 dtex and to attach such elastic material to the free edge portion 52 after stretched from the relaxed or contracted state at a stretch ratio ranging about 1.2 to about 1.5.

The free edge portion 52 of the barrier-flap 50 includes a contractile region 54 in which the flap elastic element 53 is attached under tension but in a contractible manner and an inelastic region (or low-elastic region) 55 in which the flap elastic element 53 is arranged in a substantially inelasticized state (See Figs. 9 and 10). The barrier-flap 50 partially overlaps with the first and second cover sheets 61, 62 and the inelastic region 55 is fixed to the cover sheet 60 through the intermediary of both fixed lateral portions 66, 67 described below. In the urine absorption pad 10 put on the wearer's body, the elastic region 54 of the free edge portion 52 contracts and, in consequence, the barrier-flap substantially arises.

The expression "substantially inelasticized" used herein means an instance in which the flap elastic element 53 is cut or removed and substantially is non-existent, an instance in which the cuff elastic element 53 is actually present in this region but expresses no contractility and when the flap elastic element 53 is really present in this region but expresses almost insensible contractility. According to different levels of the contractility of the flap elastic element 53, it is also possible to rename the elastic region 54 and the inelastic region 55 to a high elastic region and a low elastic region, respectively.

### <cover-sheets>

The first and second cover sheets 61, 62 are formed of inelastic sheet material, for example, hardly-liquid-permeable or liquid-impermeable fibrous nonwoven fabrics, air-permeable plastic films or laminate sheets thereof. When the first and second cover sheets 61, 62 are formed of fibrous nonwoven fabric, it is possible to use, for example, spun bond/melt blown/spun bond (SMS) fibrous nonwoven fabrics, spun bond fibrous nonwoven fabrics or meltblown fibrous nonwoven fabrics, each having a mass per unit area in a range of about 10 to about 30 g/m². The first and second cover sheets 61, 62 respectively have inner side edge portions (free edge portions) 61a, 62a extending the lateral direction X between the inner side edges 50a of the respective barrier-flaps 50.

The first and second cover sheets 61, 62 respectively have both fixed lateral potions 66 , 67 fixed to the respective barrier-flaps 50, central portions 68, 69 respectively located between both the lateral portions 66 on the side of the first end edge 10a and between both the lateral portions 67 on the side of the second end edge 10b, and covering portions 70, 71 respectively surrounded by both the lateral portions 66 and the central portion 68 and by both the lateral portions 67 and the central portion 69. Referring to Fig. 6, uprise of the barrier-flaps 50 causes the respective covering portions 70, 71 of the first and second cover sheets 61, 62 to be spaced apart from the topsheet 21 and consequently, first and second 3D-spaces (bodily-fluid retaining 3D-space) 72, 73 are defined between the covering portions 70, 71 and the topsheet 21, respectively.

Referring to Fig. 6, in a state of the urine absorption pad 10 placed on a flat surface, for example, a flat floor, the elastic regions 54 are let contract but rectilinearly extend in the longitudinal direction Y while the pad main body 20 is curved entirely. In the inelastic region 55, the flap elastic element 53 is in the inelasticized state and consequently a flexural region 56 is formed along a boundary between the elastic region 54 and inelastic region 55. With such particular form of the pad main body 20, a height dimension of the first and second-3D-spaces 72, 73 defined by the first and second cover sheets 61, 62 is gradually reduced from the opening toward the first and second end edges 10a, 10b.

Referring to Fig. 1, the respective inner side edges 50a of the barrier-flaps 50 cooperate with the respective inner side edges 61a, 62a of the first and second cover sheets 61, 62 to define an insertion opening 63 through which the wearer's penis is inserted. The barrier-flaps 50 uprise under contraction of the flap elastic element 53 so as to space away from the topsheet 21 and the cover sheets 60 fixed to respective upper surface sides (the side of the skin-facing surface) also space away together with the barrier-flaps 50. While the cover sheets 60 are fixed to the respective upper surfaces of the barrier-flaps 50, according to the present embodiment, to ensure that the cover sheets 60 space away together with the barrier-flaps 50, it is also possible to fix the cover sheets 60 to the under surface of the respective barrier-flaps 50, i.e., the surface facing the topsheet 21 (i.e., non-skin-facing surface). Even in such instance, it is ensured that the inner side edges 61a, 62a of the respective cover sheets 60 space away under contraction of the barrier-flaps 50 as long as the cover sheets 60 are fixed to the barrier-flaps 50.

Referring to Figs. 7 and 8, when putting the urine absorption pad 10 on the wearer's body, the wearer inserts his penis 40 through the insertion-opening 63. The scrotum 41 may be put in contact with or placed close to the upper surface of the first end region 11. With the pad 10 put on the wearer's body, the pad main body 20 is curved entirely along the wearer's body so that a receiving 3D-space S adapted to wrap around the penis may be defined by side walls formed by both the lateral regions 14 and a bottom wall formed by the intermediate region 13.

Urine discharged onto the urine absorption pad 10 put on the wearer's body is diffused into the liquid-absorbent core 23. The urine absorption pad 10 is curved along the wearer's body so that the barrier-flaps 50 may uprise under contraction of the flap elastic element 53 to form a 3D flap which functions to prevent the urine from leaking sideways. Upon the formation of the 3D-flap, the first and second cover sheets 61, 62 defining the upper surface thereof are spaced apart from the topsheet 21 to define the first and second 3D-spaces 72, 73 between the cover sheets 61, 62 and the topsheet 21. Formation of the first 3D-space 72 ensures that the flow of urine discharged from the urethral orifice is directly received by the interior surface of the second cover sheet 62 even when the tip of the penis 40 is directed upward (directed toward the side of the second end edge 10b) and, in this instance, the second 3D-space 73 functions as a (upper) leakage-barrier wall (leakage-barrier 3D-space) to prevent the urine from being discharged to the exterior of the urine absorption pad 10.

Also when the tip of the penis 40 is directed downward (directed toward the side of the first end edge 10a) and the urethral orifice faces the second end edge 11 flexed along the crotch region of the wearer, the first 3D-space 72 formed by the first cover sheet 61 ensures that the flow of urine discharged from the urethral orifice is directly received by the interior surface of the first cover sheet 61 and, in this instance, the first 3D-space 72 functions as a (lower) leakage-barrier wall (leakage-barrier 3D-space) to prevent the urine from being discharged to the exterior of the urine absorption pad 10 beyond the first end edge 10a and functions also to receive and absorb the urine having flown thereinto.

Referring to Figs. 9 and 10, the barrier-flaps 50 overlap with the first and second cover sheets 61, 62 in the previously described manner and the elastic regions 54 in the free edge portions 52 of the respective barrier-flaps 50 extend outward in the longitudinal direction Y beyond the inner side edges (edges of the opening) 61a, 62a of the first and second cover sheets 61, 62. The respective free edge portions 52 have the elastic region 54 and the inelastic region 55 in respective laminated portions 74 in which the barrier-flaps 50 overlap with the first and second cover sheets 61, 62.

In this manner, the elastic regions 54 are arranged in the respective layered portions 74 in which the respective barrier-flaps 50 overlap with the first and second cover sheets 61, 62 and, in consequence, when the barrier-flaps 50 of the pad put on the wearer's body uprise under contraction of the flap elastic element 53, the portions of the first and second cover sheets 61, 62 located on the respective upper surfaces of the barrier-flaps 61, 62 and adjacent to the inner side edges 61a, 62a (i.e., the opening-periphery) are spaced apart from the topsheet 21 in upwardly convexed shapes. In this way, compared with when the contractile force of the flap elastic element 53 does not acts on the opening periphery or compared with when the elastic regions 54 are fixed to the first and second cover sheets 61, 62 in the layered portions 74 and the contractile force thereof is reduced, a relatively large opening is formed.

If the first and second cover sheets 61, 62 are not partially but entirely elasticized, the 3D-spaces 72, 73 may be deformed until these 3D-spaces 72, 73 are closed under a force to reduce dimensions in the lateral direction X of these cover sheets 61, 62. Particularly when the first cover sheet 61 is wedged between the wearer's thighs under a force acting to reduce the dimension thereof in the lateral direction X, the covering portions 70 may be elastically deformed and, consequently, a volume of the first 3D-space 72 may be reduced. According to the present embodiment, the contractile region 54 extending along the opening periphery makes it possible to form the opening in a relatively large size and the inelastic regions 55 located on both sides of the respective covering portions 70, 71 function to prevent the covering portions 70, 71 from being deformed. In this way, it is possible to form the 3D-spaces 72, 73 each stably having a desired volume.

As previously described, the pad main body 20 has a shape gradually tapered from the second end edge 10b to the first end edge 10a so that the pad main body 20 may be curved toward the skin facing surface side of the intermediate region 13 under a contractile force of the elastic region 54 more easily in the first end region 11 than in the second end region 12. For this reason, it is possible for the pad main body 20 to absorb the amount of urine flowing along the surface of the topsheet 21 into the second 3D-space 73. More specifically, a dimension in the lateral direction X of the barrier-flap 50 in the first end region 11 (i.e., a distance dimension in the lateral direction X between the inner side edge 50a of the barrier-flap 50 and the associated linear sealing portion 18) is smaller than that in the second end region 12 and, for this reason, the contractile force of the elastic region 54 effectively acts on a range of the first end region 11 extending to both the side edges 10c, 10d along which the linear sealing portion 18 extends, making it possible to curve the first end region 11. In the second end region 12, the distance dimension is relatively large and, for this reason, the contractile force of the elastic region 54 hardly acts on the vicinity of the linear sealing portions, making it impossible to curve the second end region 12 like the first end region 11.

The first and second end regions 11, 12 are flexed (curved) toward the skin-facing surface side in this manner and a dimension in the longitudinal direction Y of the elastic region 54 (i.e., the distance dimension in the longitudinal direction Y between both the side edge portions 66, 67) L2 is reduced. The cover sheets 60 are spaced apart upward under the contraction of the elastic region 54 and, in consequence, a distance dimension between the topsheet 21 and the respective cover sheets 60 is correspondingly enlarged so as to enlarge respective openings of the first and second 3D-spaces 72, 73.

### <extensibility>

In the free edge portion 52, a ratio of the extensibility of the elastic region 54 per a given width to the extensibility of the inelastic region (or the low elatic region) is in a range of about 1.1 to 1.8. If this ratio is less than 1.1, even when the barrier-flaps 50 uprise in the pad put on the wearer's body, the first and second cover sheets 61, 62 may insufficiently space away from the topsheet 21 to form the respective openings of desired sizes. Meanwhile, if the ratio exceeds 1.8, the barrier-flaps 50 may more remarkably contract in the longitudinal direction Y so that the dimension in the longitudinal direction Y of the respective barrier-flaps 50 may be excessively reduced. Consequently, contours of the respective insertion openings may be scaled down and uncomfortably tighten up the penis inserted through the insertion opening 63.

Now various dimensions of the sheets 60 will be described. Length dimensions L3, L4 in the longitudinal direction Y of the first and second cover sheets 61, 62 ranges about 1/10 to about 1/3 of the dimension L1 in the longitudinal direction Y of the pad main body 20. In the present embodiment, the dimension L3 in the longitudinal direction Y of the first cover sheet 61 is larger than the dimension L4 in the longitudinal direction Y of the second cover sheet 62, specifically, the former being about 80 mm and the latter being about 40 mm.

A dimension L5 in the longitudinal direction Y of the covering portion 70 of the first cover sheet 61 is about 40 mm and ranges about 5 to about 35% of the dimension L1 in the longitudinal direction Y of the pad main body 20. If less than 5%, the dimension L5 may be insufficient to form the opening and if larger than 35%, when the urine is retained in the first 3D-space 72 to a permissible limit, the fixation effect of the central fixed portion 68 may be no more assured and the urine leakage may occur. A length dimension L6 in the longitudinal direction Y of the elastic region 54 in the laminated portion 74 of the first cover sheet 61 is about 10% of the dimension L3 in the longitudinal direction Y of the first cover sheet 62. A dimension L7 in the longitudinal direction Y of the covering portion 71 of the second cover 62 is about 20 mm, i.e., in a range of about 5 to about 35% of the dimension L1 in the longitudinal direction Y of the pad main body 20. A dimension L8 in the longitudinal direction Y of the contractile region 54 in the layered portion 74 of the second cover sheet 62 is about 12.5% of the dimension L4 in the longitudinal direction Y of the second cover sheet 62.

The respective dimensions L5, L7 in the longitudinal direction Y of the covering portions 70, 71 of the first and second cover sheets 61, 62 significantly affect volumes of the first and second 3D-spaces 72, 73, particularly on dimensions thereof in the longitudinal direction Y (i.e., depth dimensions measured from the opened edge to the closed edge in each of the first and second 3D-spaces). For example, when the dimensions L5, L7 in the longitudinal direction Y of the respective covering portions 70, 71 of the first and second cover sheets 61, 62 are about 5% or more of the dimension L1 in the longitudinal direction Y of the pad main body 20, relatively large volumes for the first and second 3D-space 72, 73 are ensured. Specifically, in the pad put on the wearer's body, the upper and lower leakage-barrier walls both having sufficiently large dimensions are formed to prevent the urine from leaking outward and, in addition, it is possible for the first 3D-space 72 to retain a certain amount of urine. When the dimensions L5, L7 in the longitudinal direction Y of the covering portions 70, 71 of the first and second cover sheets 61, 62 are about 37.5% or more of the dimension L1 in the longitudinal direction Y of the pad main body 20, a volume of the receiving 3D-space S inclusive of the first and second 3D-spaces 72, 73 becomes too large to ensure a desired fit for the penis 40 and a gap may be left between the pad main body 20 and the penis 40. The dimensions L6, L8 in the longitudinal direction Y of the contractile region 54 in the laminated portion 74 are preferably 50% or less of the dimensions L5, L7 in the longitudinal direction Y of the covering portions 70, 71 in the first and second cover sheets 61, 62 for the reason that, if the dimensions L6, L8 exceed 50% of L5, L7 in the pad put on the wearer's body, the inner side edges 61a, 62a further uprise convexly so as to enlarge the openings and an occupancy of the contractile region 54 in the laminated portions 74 becomes relatively high. This means a possibility that the cover sheet 60 may have elasticity, could no more resist external force applied to the cover sheet 60 in the lateral direction X and may be elastically deformed.

Referring again to Fig. 3, the liquid-absorbent core 23 has a shape gradually tapered from the second end region 12 toward the first end region 11 in the similar fashion for the outer shape of the pad main body 20 but at a taper ratio higher than that for the pad main body 20. Consequently, distance R1, R2 in the lateral direction X between the side edge 23c of the liquid-absorbent core 23 and the side edges 10c, 10d of the pad main body 20 are related to each other so that the distance R1 on the side adjacent to the first end region 11 is larger than the distance dimension R2 on the side adjacent to the second end region 12. The urine-absorption pad 10 put on the wearer's body is curved and the side walls located to wrap around the penis are formed by both the lateral regions 14 of the urine absorption pad 10 wherein a height of these side walls corresponds to the distance dimensions R1, R2 in the lateral direction X extending from the side edges of the urine-absorbent core 23 to both the side edges 10c, 10d of the urine absorption pad 10. In this manner, the side wall formed in the first end region 11 has a vertical dimension higher than the side wall formed in the second region 12, ensuring it to receive the penis as if wrapped around from underneath.

A stiffness value of the first and second cover sheets 61, 62 is higher than that of the barrier-flap 50. Specifically, a bending resistance of the cover sheets 60 measured on the basis of cantilever method is about 70 mm and a bending resistance of the barrier-flaps 50 measured on the basis of the cantilever method is 40 mm. A ratio of the bending resistance of the cover sheets 61, 62 to the bending resistance of the barrier-flaps 50 is in a range of about 1.5 to about 3.0.

The stiffness of the cover sheets 60 is higher than that of the barrier-flaps 50 and, in consequence, it is ensured that, even when the urine-absorption pad 10 is subjected to an external force intending to contract the pad 10 in the lateral direction X, the barrier-flaps 50 may be somewhat deformed under such external force but the cover sheets 60 well resist such external force without being deformed, thereby preventing the insertion openings 63 from being reduced in size. In addition, fixation of the barrier-flaps 50 to the cover sheets 60 having the stiffness higher than that of the former makes it possible to weaken further a remaining contractile force of the inelastic regions 55 in the respective fixed side edge portion 66, 67, thereby preventing the barrier-flaps 50 from being peeled off from both the fixed side edge portion 66 under a contractile force of the flap-elastic elements 53.

As previously described, the ratio of the stiffness represented in the cantilever bending resistance value of the cover sheets 60 to that of the barrier-flaps 50 is in the range of about 1.5 to about 3.0. Concerning such correlation of the stiffness values, if a ratio of a stiffness value of the former to that of the latter is about 1.5 or less, when the former is subjected to an external force in the lateral direction X sufficient to deform the latter, the former may be flexed and deformed to reduce the size of the opening. If a ratio of a stiffness value of the former to that of the latter is about 3.0 or more, a stiffness value of the former may become excessively high and the wearer exposed thereto may experience a sense of discomfort and/or suffer from skin trouble caused by irritation.

### <measuring method for cantilever bending resistance>

In conformity to the cantilever method prescribed in JIS L 1096, test pieces (150 mm in the direction X, 25 mm in the direction Y) were punched out from the used for the barrier-flap 50 and the cover sheet 60, respectively, and the measurement was conducted on the skin-facing surface and the non-skin-facing surface for each of the test pieces. A movement rate of the sheet was 5 mm/sec. The bending resistance was measured once on the skin-facing surface and once on the non-skin-facing surface for each of the test pieces the measured values on these two surfaces were averaged to obtain the bending resistance value (mm) of the respective sheets.

Although the cover sheets 60 are located in both the end regions as viewed in the longitudinal direction Y of the pad main body 20, respectively, according to the present invention as well as according to the other embodiments described below, the present invention is not limited to such arrangement and it is also possible to locate the cover sheet 60 in only one of the first and second end regions as viewed in the longitudinal direction Y. In addition, the string-like flap-elastic elements 53 are attached under tension but in a contractible manner to the respective free edge portions 52 of the barrier-flaps 50 to elasticize the barrier-flaps 50 according to these embodiments, the present invention is not limited to such arrangement and it is also possible, for example, to attach a fibrous nonwoven fabric sheet which is extensible/contractible in the longitudinal direction Y to the barrier-flaps 50. When two sheets are fixed to each other, it is possible, for example, to coat at least one of the sheets with hot melt adhesive, thereby fixing these two sheets with the hot melt adhesive or, if the sheets are thermoplastic fibrous nonwoven fabric sheets, it is also possible to heat-bond them to each other.

The expression "the contractile action of the contractible region is exerted to the peripheries of the respective openings" used in the specification of the present invention includes, as previously described, includes three instances as follows: (1) the instance in which the elastic region 64 extends to the portion of the barrier-flap 50 layered with the cover sheet 60; (2) the instance in which the end portion of the elastic region 54 falls into line with a boundary 59 between the barrier-flap 50 and the associated cover sheet 60 illustrated in Fig. 11; and (3) the instance in which the end portion of the elastic region 54 extends not to the layered portion 74 of the barrier-flap 50 and the associated cover sheet 60 but to the vicinity of the boundary 59, more specifically, to a portion in which only the barrier-flap 50 is present, as illustrated in Fig. 3.

In the instance (3) described above, it is required to intensify the contractile force of the flap-elastic element 53 compared to the instances (1) and (2) in order to assure the above set forth advantageous function and effect of the present invention. For example, it is required to use an elastic material having a fineness (string diameter) of about 1240 dtex as the flap-elastic element 53 and to attach such elastic material to the barrier-flap under extension at a stretch ration in a range of about 1.5 to about 2.0 from a contractile or natural state. In other words, it is necessary in the instance (3) to use the elastic material thicker than those used in the instances (1) and (2) as the flap-elastic element 53 and to fix the flap-elastic element 53 to the barrier-flap under extension higher than in the instances (1) and (2). In the instance (3), a dimension L9 in the longitudinal direction Y between the boundary 59 and the end portion of the elastic region 54 is in a range of 0 mm to about 15 mm. In this manner, not only when the elastic region 54 extends to the boundary 59 as in the instance (2) but also when the elastic region 54 extends not to the boundary 59 as in the instance (3), a contractile force of the elastic region 54 substantially acts on the peripheral edge portion of the opening, making it possible to draw up the peripheral edge portion of the opening. To ensure such effect in the instance (3), it is essential to use the flap-elastic element 53 having a relatively high contractility and to set the dimension L9 between the end portion of the elastic region 54 and the boundary 59 in the above-mentioned relatively limited range. According to the present embodiment corresponding the instance (1), the elastic region 54 extending to the peripheral edge portion of the opening makes it possible to draw up the peripheral edge portion of the opening and, also according to the embodiments corresponding to the instances (2) and (3), the contractile force of the contractile region 54 substantially acts on the peripheral edge portion and thereto to outspread the opening.

### <Second Embodiment>

According to this embodiment illustrated in Fig. 13, in the intermediate region 13, the barrier-flaps 50 are fixed to the pad main body 20 not only along the sealing portion 18 but also along proximal fixed portions 79 linearly extending along the side edges 10c, 10d. Such arrangement that, in the intermediate region 13, the barrier-flaps 50 are fixed to the pad main body 20 along the sealing portion 18 and the proximal fixed portions 79 inevitably limits the height of the 3D flaps but improves a peel resistance of the barrier-flaps 50. The width dimension of the respective proximal fixed portions 79 tapers from the side of the second end region 12 toward the side of the first end region 11. Consequently, a height dimension of the respective 3D flaps is larger on the side of the second end region 12 than on the side of the first end region 11 so as to improve the peel resistance and to prevent leakage of the urine from above reliably.

### <Third Embodiment>

According to this embodiment illustrated in Fig. 14, flexion-inducing grooves 80 are arranged in a portion of the intermediate region 13 of the urine absorption pad 10 not covered with the barrier-flaps 50 and the first and second cover sheets 61, 62. The flexion-inducing grooves 80 are recess lines compressed from the topsheet 21 toward the liquid-absorbent core 23 and include a first groove 81 obliquely intersecting with the longitudinal axis P, a second groove 82 extending in a direction intersecting with the first groove 81 and third grooves 83 wherein the third grooves 83 include a first segment 83A located closer to the first end edge 10a compared with the first and second grooves 81, 82 so as to extend on the longitudinal axis P and second segments 83B located symmetrically about the longitudinal axis P and obliquely intersecting with the longitudinal axis P. According to this embodiment, the first and second grooves 81, 82 are discontinuously formed, more specifically, none of real groove segments are formed in the crossover region of the first and second grooves 81, 82.

The intermediate region 13 of the urine absorption pad 10 is convexly deformed outward (toward the side of the clothes) under the effect of the flexion-inducing grooves 80. Such deformation makes it possible, more reliably than the instance having none of the flexion-inducing grooves, to form the receiving 3D-space S of cup-like shape adapted to receive the penis as if the side walls and the bottom wall defining the convexly deformed region wrap around the penis. In this manner, the intermediate region 13 defines the convexly deformed region to be formed of the cup-like shape so stabilized that the first and second end regions 11, 12 may be flexed toward the wearer's body and the dimension of the elastic region 54 may be correspondingly reduced. In consequence, the barrier-flaps 50 further uprise and the openings of the first and second 3D-spaces 72, 73 are further outspreaded. Particularly, the third grooves 83 extend to the vicinity of the inner side edge 61a of the first cover sheet 61 and, when the first end region 11 is flexed through the third grooves 83, the inner side edge 61a get relatively closer to the transverse axis Q and the opening of the first 3D-space 72 is formed in a relatively large size. Further, the flexion-inducing grooves 80 formed in the liquid-absorbent core 23 as has been described above ensure that the urine discharged is diffused through the intermediary of the flexion-inducing grooves 80 over the entirety of the liquid-absorbent core 23 and thereby an absorption velocity is improved.

### <Fourth Embodiment>

According to this embodiment illustrated in Fig. 15, the urine absorption pad 10 has a pair of fold lines 85, 86 extending in the lateral direction X. The fold lines 85, 86 are formed in consequence of storing the urine absorption pad 10 in its folded state or individually packaging the pad in its folded state in an envelope for this purpose and, even when the urine absorption pad 10 is in its flatten state (or in natural state), a trace of the fold line is normally left. In the pad put on the wearer's body, the intermediate region 13 is flexed along the flexion lines 85, 86 and a deformed region convexed toward the side of the garment is formed between the flexion lines 85, 86. The first end region 11 and the second end region 12 are flexed along the flexion lines 85, 86 and, in consequence, the dimension L2 in the longitudinal direction Y of the elastic region 54 is reduced and correspondingly the barrier-flaps 50 further uprise. In consequence, the respective openings of the first and second 3D-spaces 72, 73 are outspreaded. As long as such advantageous effect is ensured, at least one of the fold lines 85, 86 may be omitted.

### <Fifth Embodiment>

According to this embodiment illustrated in Fig. 16, a thickness dimension of the liquid-absorbent core 23 in the first and second end regions 11, 12 is larger than that in the intermediate region 13. The portions of the liquid-absorbent core 23 covered with the first and second cover sheets 61, 62, respectively, may be dimensioned to be thicker than the intermediate region 13 in this manner to define a pair of high-absorbency regions, thereby assuring that any amount of the urine which otherwise would leak out from the receiving 3D-space is reliably absorbed and retained. Specifically, a mass per unit area of a thinner portion 90 of the liquid-absorbent core 23 defined in the intermediate region 13 is about 400 g/m² and a mass per unit area of the respective thicker portions 91 defined in the first and second end regions 11, 12, respectively, is about 500 g/m². Although the stiffness of the liquid-absorbent core 23 changes at respective boundaries 92 between the a thin portion 90 and respective thick portions 91, i.e., the portions in which the thickness dimension of the liquid-absorbent core 23 changes, the openings of the first and second cover sheets 61, 62 are located in the vicinity of the boundaries 92, respectively, and the dimension L2 of the elastic region 54 is reduced as the first and second end regions 11, 12 are flexed along the respective boundaries 92. In consequence, the barrier-flaps 50 further uprise and the openings are further outspreaded.

### <Sixth Embodiment>

According to this embodiment illustrated in Fig. 17, a width dimension of the sealing portion 18 is gradually enlarged from the intermediate region 13 to the first end edge 10a, specifically, a width dimension W3 of the sealing portion 18 in the first end edge 10a is larger than a width dimension W2 thereof in the intermediate region 13. The sealing portion 18 may be arranged in the relation of the dimension W3 > W2 to ensure that, even if the urine is retained within the first 3D-space to an acceptability limit until the covering portion 70 swells and the central fixed potion 68 is peeled off, the first 3D-space 72 is stably sealed by the sealing portion 18 in the first end edge 10a to prevent the urine from leaking out. Though not illustrated, the central fixed portion 68 may be further enlarged until this portion 68 become continuous with the sealing portion 18 along the first end edge 10a facing the portion 68 in the longitudinal direction Y to improve a sealing strength.

The disclosure relating to the present invention described above may be arranged at least as follows:
(1) A male urine absorption pad having a longitudinal direction, a lateral direction being orthogonal thereto, a skin-facing surface and a non-skin-facing surface opposed thereto and including a pad main body having a liquid-absorbent core. The pad main body has first and second end regions spaced apart from and facing to each other in the longitudinal direction, both lateral regions spaced apart from and facing each other and a central region defined between both the lateral regions. Of the first and second end regions, at least the first end region is provided with a cover sheet adapted to cover the central region and both the lateral regions are provided with a pair of barrier-flaps extending in the longitudinal direction. Each of the barrier-flaps has both end portions fixed to the skin-facing surface side of the pad main body and free edge portion extending in the longitudinal direction between both the fixed end portions. Each of the free edge portions has an elastic region being contractible in the longitudinal direction and inelastic regions located on the outer side of the elastic region as viewed in the longitudinal direction. The cover sheet is formed of inelastic sheet material and has inner edge portion extending across the central region, both side edge portions fixed to the barrier-flap in the first end region, a central portion fixed to the pad main body located between both the fixed side edge portions and a covering portion defined between both the fixed side edge portions and the central fixed portion to cover the central region. A urine-receivable 3D-space having an opening-peripheral edge portion defined by the inner side edge portion of the cover sheet is formed between the covering portion and the pad main body; and a contractile action of the elastic region is exerted to the opening-peripheral edge portion.
(2) A male urine absorption pad having a longitudinal direction, a lateral direction being orthogonal thereto, a skin-facing surface and a non-skin-facing surface opposed thereto and including a pad main body having a liquid-absorbent core. The pad main body has first and second end regions spaced apart from and facing to each other in the longitudinal direction, both the lateral regions spaced apart from and facing each other and a central region defined between both the lateral regions. Of the first and second end regions, at least the first end region is provided with a cover sheet adapted to cover the central region and both the lateral regions are provided with a pair of barrier-flaps extending in the longitudinal direction; each of the barrier-flaps has both end portions fixed to the skin-facing surface side of the pad main body and free edge portion extending in the longitudinal direction between both the fixed end portions. Each of the free edge portions has an elastic region being contractible in the longitudinal direction and inelastic regions located on the outer side of the elastic region as viewed in the longitudinal direction. The cover sheet is formed of inelastic sheet material and has inner edge portion extending across the central region, both side edge portions fixed to the barrier-flap in the first end region, a central portion fixed to the pad main body located between both the fixed side edge portions and a covering portion defined between both the fixed side edge portions and the central fixed portion to cover the central region; a urine-receivable 3D-space having an opening-peripheral edge portion defined by the inner side edge portion of the cover sheet is formed between the covering portion and the pad main body, and the contractile region is defined between at least one fixed end on the side of the second end region and the fixed side edge portion.

The present invention disclosed in the paragraph {0058} may include embodiments at least as described below and these embodiments may be taken in isolation or in combination.
(1) The elastic region extends to a portion of the barrier-flap layered with the cover sheet and the inelastic region is fixed to both the fixed side edge portion of the cover sheet.
(2) The cover sheet is fixed to the skin-facing surface side of the barrier-flap.
(3) The pad main body and the barrier-flap have respective shapes tapered from the second end region toward the first end region.
(4) The pad main body is additionally provided with a second cover sheet fixed to the barrier-flap in the second end region.
(5) The cover sheet has a stiffness value based on a cantilever bending resistance higher than that of the barrier-flap.
(6) The barrier-flap is provided in the elastic region thereof with a linear elastic element attached thereto.
(7) The pad main body further includes an intermediate region defined in the longitudinal direction between the first end region and the second end region, a liquid-permeable topsheet located on the skin-facing surface side, and flex-inducing grooves arranged between the inner side edges of the barrier-flap in the intermediate region to recess the topsheet and the liquid-absorbent core from the skin-facing surface side toward the non-skin-facing surface side.
(8) A dimension in the longitudinal direction of the covering portion of the cover sheet is about 5 % or more of a dimension in the longitudinal direction of the pad main body and a dimension in the longitudinal direction of the contractile region is about 50 % or less of the dimension in the longitudinal direction of the covering portion.
(9) At least in the first end region of the first and second end regions, the liquid-absorbent core is thicker than the intermediate region and there is a portion in the vicinity of a boundary between the first end region and the intermediate region, in which the thickness dimension of the liquid-absorbent core changes and the opening's peripheral edge is defined so as to face the portion in which the thickness dimension changes.
(10) The male urine-absorption pad further includes at least one fold line extending in the lateral direction and intersecting with the barrier-flap but not with the cover sheet.
(11) The liquid-absorbent core is shaped so as to be tapered from the second end region toward the first end region and a distance dimension in the lateral direction between respective side edges of the liquid-absorbent core and respective side edges of the pad main body in the first end region is larger than a distance dimension between side edges of the liquid-absorbent core and side edges of the pad main body in the second end region.
(12) The barrier-flaps further have proximal fixed portions extending along both side edges of the pad main body and the respective proximal fixed portions have a width dimension larger on the side of the second end region than on the side of the first end region.

### {Reference Signs List}

- 10: urine absorption pad
- 11: first end area
- 12: second end area
- 13: intermediate area
- 14: both lateral areas
- 15: central area
- 20: pad main body
- 21: topsheet
- 23: liquid-absorbent core
- 50: barrier-flaps
- 53: flap elastic element (elastic element)
- 54: elastic region
- 52: free edge portion
- 55: inelastic region
- 58: fixed both end portions
- 61: first cover sheet
- 62: second cover sheet
- 61a: inner side edge of cover sheet
- 62a: inner side edge of cover sheet
- 66: fixed side edge portion
- 67: fixed side edge portion
- 68: central fixed portion
- 74: layered portion
- 75: elastic region (high-elastic region)
- 76: inelastic region (low-elastic region)
- 79: proximal fixed portion
- 80: flexion-inducing groove
- X: lateral direction
- Y: longitudinal direction
- Z: thickness direction

## Claims

1. A male urine absorption pad (10) having a longitudinal direction (Y), a lateral direction (X) being orthogonal thereto, a skin-facing surface and a non-skin-facing surface opposed thereto and including a pad main body (20) having a liquid-absorbent core (23), **characterized in that**:
the pad main body (20) has first and second end regions (11, 12) spaced apart and facing to each other in the longitudinal direction (Y), both lateral regions (14) spaced apart from and facing each other and a central region (15) defined between both the lateral regions (14);
of the first and second end regions (11, 12), at least the first end region (11) is provided with a cover sheet (61) adapted to cover the central region (15) and both the lateral regions (14) are provided with a pair of barrier-flaps (50) extending in the longitudinal direction;
each of the barrier-flaps (50) has both end portions (58) fixed to the skin-facing surface side of the pad main body (20) and free edge portion (52) extending in the longitudinal direction (Y) between both the fixed end portions (58);
each of the free edge portions (52) has an elastic region (54) being contractible in the longitudinal direction (Y) and inelastic regions (55) located on the outer side of the elastic region (54) as viewed in the longitudinal direction (Y);
the cover sheet (6) is formed of inelastic sheet material and has inner edge portion (61a) extending across the central region (15), both side edge portions (66) fixed to the barrier-flap (50) in the first end region (11), a central portion (68) fixed to the pad main body (20) located between both the fixed side edge portions (66) and a covering portion (70) defined between both the fixed side edge portions (66) and the central fixed portion (68) to cover the central region (15);
an urine-receivable 3D-space (72) having an opening-peripheral edge portion defined by the inner side edge portion (61a) of the cover sheet (61) is formed between the covering portion (70) and the pad main body (20); and
a contractile action of the elastic region (75) is exerted to the opening-peripheral edge portion.

2. The urine absorption pad (10) according to Claim 1 wherein the elastic region (54) extends to a portion of the barrier-flap (50) layered with the cover sheet (61); and
the inelastic region (55) is fixed to both the fixed side edge portion (66) of the cover sheet (61).

3. The urine absorption pad (10) according to Claim 1 or 2 wherein the cover sheet (61) is fixed to the skin-facing surface side of the barrier-flap (50).

4. The urine absorption pad (10) according to any one of Claims 1 through 3 wherein the pad main body (20) and the barrier-flap (50) have respective shapes tapered from the second end region (12) toward the first end region (11).

5. The urine absorption pad (10) according to any one of Claims 1 through 4 wherein the pad main body (20) is additionally provided with a second cover sheet (62) fixed to the barrier-flap (50) in the second end region (12).

6. The urine absorption pad (10) according to any one of Claims 1 through 5 wherein the cover sheet (61, 62) has a stiffness value based on a cantilever bending resistance higher than that of the barrier-flap (50).

7. The urine absorption pad (10) according to any one of Claims 1 through 6 wherein the barrier-flap (50) is provided in the elastic region (55) thereof with a linear elastic element (53) attached thereto.

8. The urine absorption pad (10) according to any one of Claims 1 through 7 wherein the pad main body (20) further includes an intermediate region (13) defined in the longitudinal direction (Y) between the first end region (11) and the second end region (12), a liquid-permeable topsheet (21) located on the skin-facing surface side, and flexion-inducing grooves (80) arranged between the inner side edges (50a) of the barrier-flap (50) in the intermediate region (15) to recess the topsheet (21) and the liquid-absorbent core (23) from the skin-facing surface side toward the non-skin-facing surface side.

9. The urine absorption pad (10) according to any one of Claims 1 through 8 wherein a dimension (L5, L7) in the longitudinal direction (Y) of the covering portion (70, 71) of the cover sheet (61, 62) is about 5 % or more of a dimension (L1) in the longitudinal direction (Y) of the pad main body (20) and a dimension (L6) in the longitudinal direction (Y) of the elastic region (54) is about 50 % or less of the dimension (L3) in the longitudinal direction of the covering portion (70, 71).

10. The urine absorption pad (10) according to Claim 8 or 9 wherein, at least in the first end region (11) of the first and second end regions (11, 12), the liquid-absorbent core (23) is thicker than the intermediate region (13) and there is a portion in the vicinity of a boundary (92) between the first end region (11) and the intermediate region (13), in which the thickness dimension of the liquid-absorbent core (23) changes and the opening's peripheral edge is defined so as to face the portion in which the thickness dimension changes.

11. The urine absorption pad (10) according to any one of Claims 1 through 10, further including at least one fold line (85, 86) intersecting with the barrier-flap (50) but not with the cover sheet (61, 62).

12. The urine absorption pad (10) according to Claim 4 wherein the liquid-absorbent core (23) is shaped so as to be tapered from the second end region (12) toward the first end region (11) and a distance dimension (R1) in the lateral direction between respective side edges of the liquid-absorbent core (23) and respective side edges (10c, 10d) of the pad main body (20) in the first end region (11) is larger than a distance dimension (R2) between side edges of the liquid-absorbent core (23) and side edges (10c, 10d) of the pad main body (20) in the second end region (12).

13. The urine absorption pad (10) according to any one of Claims 8 through 12 wherein the barrier-flaps (50) further have proximal fixed portions (79) extending along both side edges (10c, 10d) of the pad main body (20) and the respective proximal fixed portions (79) have a width dimension larger on the side of the second end region (12) than on the side of the first end region (11).

## Patentansprüche

1. Ein Männerurinabsorptionskissen (10) mit einer longitudinalen Richtung (Y), einer lateralen (X) Richtung, die dazu orthogonal ist, eine Haut-zugewandte Oberfläche und eine nicht-Haut-zugewandte Oberfläche, die dazu gegenüber liegt und einen Kissenhauptkörper (20) mit einem flüssigkeitsabsorbierenden Kern (23) umfasst, **dadurch gekennzeichnet, dass**:
der Kissenhauptkörper (20) erste und zweite Endbereiche (11, 12), die voneinander beabstandet und in der longitudinalen Richtung (Y) einander zugewandt sind, beide Seitenbereiche (14), die voneinander beabstandet und einander zugewandt sind, und einen Zentralbereich (15), der zwischen den beiden Seitenbereichen (14) bestimmt ist, aufweist;
von den ersten und zweiten Endbereichen (11, 12), wenigstens der erste Endbereich (11) über eine Decklage (61), ausgelegt um den Zentralbereich (15) zu bedecken, verfügt und beide Seitenbereiche (14) über ein Paar Barriere-Klappen (50), die sich in der longitudinalen Richtung erstrecken, verfügen;
jede der Barriere-Klappen (50) beide Endteile (58), die an der Haut-zugewandten Oberflächenseite des Kissenhauptkörpers (20) und freien Randteils (52), der sich in der longitudinalen Richtung (Y) zwischen beiden der befestigten Endteile (58) erstreckt, befestigt sind:
jeder der freien Randteile (52) einen elastischen Bereich (54), der zusammenziehbar in der longitudinalen Richtung (Y) ist, und unelastische Bereiche (55), die sich in der longitudinalen Richtung (Y) gesehen auf der Außenseite des elastischen Bereichs (54) befinden, aufweist;
die Decklage (6) aus unelastischem Lagenmaterial gebildet ist und eine inneren Randteil (69a), der sich über den Zentralbereich (15) erstreckt, beide Seitenwandteile (66), die an der Barriere-Klappe (50) in dem ersten Endbereich (11) befestigt sind, einen Zentralteil (68), der an dem Kissenhauptkörper (20), der sich zwischen beiden der befestigten Seitenrandteilen (66) befindet, befestigt ist, und den zentral befestigten Teil (68), um den Zentralbereich (15) zu bedecken, aufweist;
ein urinaufnehmbarer 3D-Raum (72) mit einem Öffnungsaußenrandteil, der durch den inneren Seitenrandteil (61a) der Decklage (61) bestimmt ist, zwischen dem Deckteil (70) und dem Kissenhauptkörper (20) ausgebildet ist; und
eine zusammenziehende Bewegung des elastischen Bereichs (75) auf den Öffnungsaußenrandteil ausgeübt wird.

2. Das Urinabsorptionskissen (10) gemäß Anspruch 1 wobei sich der elastische Bereich (54) zu einem Teil der Barriere-Klappe (50), die mit der Decklage (61) bedeckt ist, erstreckt; und
der unelastische Teil (55) an beide der befestigten Seitenrandteile (66) der Decklage (61) befestigt ist.

3. Das Urinabsorptionskissen (10) gemäß Anspruch 1 oder 2, wobei die Decklage (61) an die Haut-zugewandte Oberflächenseite der Barriere-Klappe (50) befestigt ist.

4. Das Urinabsorptionskissen (10) gemäß einem der Ansprüche 1 bis 3, wobei der Kissenhauptkörper (20) und die Barriere-Klappe (50) jeweilige Formen aufweisen, die sich von dem zweiten Endbereich (12) zu dem ersten Endbereich (11) zuspitzen.

5. Das Urinabsorptionskissen (10) gemäß einem der Ansprüche 1 bis 4, wobei der Kissenhauptkörper (20) zusätzlich mit einer zweiten Decklage (62), die an der Barriere-Klappe (50) in dem zweiten Endbereich (12) befestigt ist, versehen ist.

6. Das Urinabsorptionskissen (10) gemäß einem der Ansprüche 1 bis 5, wobei die Decklage (61, 62) einen Steifigkeitswert, basierend auf einer freitragenden Biegesteifigkeit höher als die der Barriere-Klappte (50), aufweist.

7. Das Urinabsorptionskissen (10) gemäß einem der Ansprüche 1 bis 6, wobei die Barriere-Klappe (50) in dem elastischen Bereich (55) davon mit einem daran befestigten linear elastischen Element (53) vorgesehen ist.

8. Das Urinabsorptionskissen (10) gemäß einem der Ansprüche 1 bis 7, wobei der Kissenhauptkörper (20) ferner einen Zwischenbereich (13), der in der longitudinalen Richtung (Y) zwischen dem ersten Endbereich (11) und dem zweiten Endbereich (12) bestimmt ist, eine flüssigkeitsdurchlässige Oberlage (21), die auf der Haut-zugewandten Oberflächenseite angeordnet ist, und beugungs-einleitende Nuten (80), die zwischen dem inneren Seitenrand (50a) der Barriere-Klappe (50) in dem Zwischenbereich (15) angeordnet sind um die Oberlage (21) und den flüssigkeitsabsorbierenden Kern (23) von der Haut-zugewandten Oberfläche zur nicht-Haut-zugewandten Oberflächenseite auszusparen, umfasst.

9. Das Urinabsorptionskissen (10) gemäß einem der Ansprüche 1 bis 8, wobei eine Dimension (L5, L7) in der longitudinalen Richtung (Y) des Deckteils (70, 71) der Decklage (61, 62) ungefähr 5 % oder mehr einer. Dimension (L2) in der longitudinalen Richtung (Y) des Kissenhauptkörpers (20) beträgt und eine Dimension (L6) in der longitudinalen Richtung (Y) des elastischen Bereichs (54) ungefähr 50 % oder weniger der Dimension (L3) in der longitudinalen Richtung des Deckteils (70, 71) beträgt.

10. Das Urinabsorptionskissen (10) gemäß Anspruch 8 oder 9, wobei, wenigstens in dem ersten Endbereich (11) der ersten und zweiten Endbereiche (11, 12), der flüssigkeitsabsorbierende Kern (23) dicker als der Zwischenbereich (13) ist und es einen Teil in der Umgebung einer Grenze (92) zwischen dem ersten Endbereich (11) und dem Zwischenbereich (13) gibt, wobei sich die Dickendimension des flüssigkeitsabsorbierenden Kerns (23) ändert und der Umfangsrand der Öffnung bestimmt ist, um dem Teil zugewandt zu sein, in dem sich die Dickendimension ändert.

11. Das Urinabsorptionskissen (10) gemäß einem der Ansprüche 1 bis 10, ferner wenigstens eine gefaltete Linie (85, 86) umfassend, die die Barriere-Klappe (50) aber nicht die Decklage (61, 62) schneidet.

12. Das Urinabsorptionskissen (10) gemäß Anspruch 4, wobei der flüssigkeitsabsorbierende Kern (23) ausgeformt ist, um konisch von dem zweiten Endbereich (12) in Richtung des ersten Endbereichs (11) zu sein, und eine Abstandsdimension (R1) in der lateralen Richtung zwischenentsprechenden Seitenrändern des flüssigkeitsabsorbierenden Kerns (23) und entsprechenden Seitenrändern (10c, 10d) des Kissenhauptkörpers (20) in dem ersten Randbereich (11) größer als eine Abstandsdimension (R2) zwischen Seitenrändern des flüssigkeitsabsorbierenden Kerns (23) und Seitenrändern (10c, 10d) des Kissenhauptkörpers (20) in dem zweiten Endbereich (12) ist.

13. Das Urinabsorptionskissen (10) gemäß einem der Ansprüche 8 bis 12, wobei die Barriere-Klappen (50) ferner proximal befestigte Teile (79) aufweisen, die sich entlang beider Seitenränder (10c, 10d) des Kissenhauptkörpers (20) erstrecken, und die entsprechenden proximal befestigten Teile (79) eine Breitendimension aufweisen, die auf der Seite des zweiten Endbereichs (12) größer als auf der Seite des ersten Endbereichs (11) ist.

## Revendications

1. Garniture d'absorption d'urine (10) pour homme ayant une direction longitudinale (Y), une direction latérale (X) orthogonale à celle-ci, une surface tournée vers la peau et une surface non tournée vers la peau opposée à celle-ci et incluant un corps principal de garniture (20) ayant une coeur d'absorption de liquide (23), **caractérisée en ce que** :
le corps principal de garniture (20) comporte des première et seconde régions d'extrémité (11, 12) espacées l'une de l'autre et tournées l'une vers l'autre dans la direction longitudinale (Y), deux régions latérales (14) étant espacées l'une de l'autre et tournées l'une vers l'autre et une région centrale (15) définie entre les deux régions latérales (14) ;
parmi les première et seconde régions d'extrémité (11, 12), au moins la première région d'extrémité (11) est pourvue d'une feuille de recouvrement (61) adaptée pour recouvrir la région centrale (15) et les deux régions latérales (14) sont pourvues d'une paire de rabats-barrières (50) s'étendant dans la direction longitudinale ;
chacun des rabats-barrières (50) comporte deux portions d'extrémité (58) fixées au côté de surface tournée vers la peau du corps principal de garniture (20) et une portion de bord libre (52) s'étendant dans la direction longitudinale (Y) entre les deux portions d'extrémité fixées (58) ;
chacune des portions de bord libre (52) comporte une région élastique (54) pouvant se contracter dans la direction longitudinale (Y) et des régions inélastiques (55) situées sur le côté extérieur de la région élastique (54) telles que vues dans la direction longitudinale (Y) ;
la feuille de recouvrement (6) est formée d'un matériau de feuille inélastique et comporte une portion de bord intérieur (61a) s'étendant à travers la région centrale (15), les deux portions de bord latéral (66) fixées au rabat-barrière (50) dans la première région d'extrémité (11), une portion centrale (68) fixée au corps principal de garniture (20) située entre les deux portions de bord latéral fixées (66) et une portion de recouvrement (70) définie entre les deux portions de bord latéral fixées (66) et la portion fixée centrale (68) pour recouvrir la région centrale (15) ;
un espace 3D pouvant recevoir l'urine (72) ayant une portion de bord périphérique d'ouverture définie par la portion de bord latéral intérieur (61a) de la feuille de recouvrement (61) est formé entre la portion de recouvrement (70) et le corps principal de garniture (20) ; et
une action de contraction de la région élastique (75) est exercée sur la portion de bord périphérique d'ouverture.

2. Garniture d'absorption d'urine (10) selon la revendication 1 dans laquelle la région élastique (54) s'étend jusqu'à une portion du rabat-barrière (50) disposée en couche avec la feuille de recouvrement (61) ; et
la région inélastique (55) est fixée aux deux portions de bord latéral fixées (66) de la feuille de recouvrement (61).

3. Garniture d'absorption d'urine (10) selon la revendication 1 ou 2 dans laquelle la feuille de recouvrement (61) est fixée au côté de surface tournée vers la peau du rabat-barrière (50).

4. Garniture d'absorption d'urine (10) selon l'une quelconque des revendications 1 à 3 dans laquelle le corps principal de garniture (20) et le rabat-barrière (50) ont des formes respectives rétrécissant de la seconde région d'extrémité (12) vers la première région d'extrémité (11).

5. Garniture d'absorption d'urine (10) selon l'une quelconque des revendications 1 à 4 dans laquelle le corps principal de garniture (20) est en plus pourvu d'une seconde feuille de recouvrement (62) fixée au rabat-barrière (50) dans la seconde région d'extrémité (12).

6. Garniture d'absorption d'urine (10) selon l'une quelconque des revendications 1 à 5 dans laquelle la feuille de recouvrement (61, 62) présente une valeur de rigidité sur la base d'une résistance à la flexion en porte-à-faux supérieure à celle du rabat-barrière (50).

7. Garniture d'absorption d'urine (10) selon l'une quelconque des revendications 1 à 6 dans laquelle le rabat-barrière (50) est pourvu dans sa région élastique (55) d'un élément élastique linéaire (53) attaché à celui-ci.

8. Garniture d'absorption d'urine (10) selon l'une quelconque des revendications 1 à 7 dans laquelle le corps principal de garniture (20) inclut en outre une région intermédiaire (13) définie dans la direction longitudinale (Y) entre la première région d'extrémité (11) et la seconde région d'extrémité (12), une feuille supérieure perméable aux liquides (21) située sur le côté de surface tournée vers la peau, et des rainures de déclenchement de flexion (80) agencées entre les bords latéraux intérieurs (50a) du rabat-barrière (50) dans la région intermédiaire (15) pour évider la feuille supérieure (21) et le coeur d'absorption de liquide (23) du côté de surface tournée vers la peau vers le côté de surface non tournée vers la peau.

9. Garniture d'absorption d'urine (10) selon l'une quelconque des revendications 1 à 8 dans laquelle une dimension (L5, L7) dans la direction longitudinale (Y) de la portion de recouvrement (70, 71) de la feuille de recouvrement (61, 62) fait environ 5 % ou plus d'une dimension (L1) dans la direction longitudinale (Y) du corps principal de garniture (20) et une dimension (L6) dans la direction longitudinale (Y) de la région élastique (54) fait environ 50 % ou moins de la dimension (L3) dans la direction longitudinale de la partie de recouvrement (70, 71).

10. Garniture d'absorption d'urine (10) selon la revendication 8 ou 9 dans laquelle, au moins dans la première région d'extrémité (11) des première et secondes régions d'extrémité (11, 12), le coeur d'absorption de liquide (23) est plus épais que la région intermédiaire (13) et il y a une portion à proximité d'une limite (92) entre la première région d'extrémité (11) et la région intermédiaire (13), dans laquelle la dimension d'épaisseur du coeur d'absorption de liquide (23) change et le bord périphérique de l'ouverture est défini de manière à être tourné vers la portion dans laquelle la dimension d'épaisseur change.

11. Garniture d'absorption d'urine (10) selon l'une quelconque des revendications 1 à 10, incluant en outre au moins une ligne de pli (85, 86) croisant le rabat-barrière (50) mais pas la feuille de recouvrement (61, 62).

12. Garniture d'absorption d'urine (10) selon la revendications 4 dans laquelle le coeur d'absorption de liquide (23) est formé de manière à rétrécir de la seconde région d'extrémité (12) vers la première région d'extrémité (11) et une dimension de distance (R1) dans la direction latérale entre des bords latéraux respectifs du coeur d'absorption de liquide (23) et des bords latéraux respectifs (10c, 10d) du corps principal de garniture (20) dans la première région d'extrémité (11) est supérieure à une dimension de distance (R2) entre des bords latéraux du coeur d'absorption de liquide (23) et des bords latéraux (10c, 10d) du corps principal de garniture (20) dans la seconde région d'extrémité (12).

13. Garniture d'absorption d'urine (10) selon l'une quelconque des revendications 8 à 12 dans laquelle les rabats-barrières (50) ont en outre des portions fixées proximales (79) s'étendant le long des deux bords latéraux (10c, 10d) du corps principal de garniture (20) et les portions fixées proximales respective (79) ont une dimension de largeur plus grande sur le côté de la seconde région d'extrémité (12) que sur le côté de la première région d'extrémité (11).
